(19) Europäisches Patentamt
European Patent Office
Office européen des brevets

(11) Publication number: **0 125 508**
**B1**

(12) # EUROPEAN PATENT SPECIFICATION

(45) Date of publication of patent specification: **15.10.86**

(51) Int. Cl.$^4$: **A 61 K 31/265**

(21) Application number: **84104202.1**

(22) Date of filing: **13.04.84**

(54) **Method for stabilizing a detrusor muscle while increasing detrusor muscle strength.**

(30) Priority: **14.04.83 US 485056**

(43) Date of publication of application:
**21.11.84 Bulletin 84/47**

(45) Publication of the grant of the patent:
**15.10.86 Bulletin 86/42**

(84) Designated Contracting States:
**BE CH DE FR GB IT LI LU NL SE**

(56) References cited:
**US-A-2 390 555**
**US-A-2 510 773**

(73) Proprietor: **United Pharmaceuticals, Inc.**
**1500 North Wilmot Road Suite 290**
**Tucson Arizona 85712 (US)**

(72) Inventor: **Davis, William M. M.D.**
**3501 N. Bear Canyon Road**
**Tucson AZ 85712 (US)**

(74) Representative: **Weber, Dieter, Dr. et al**
**Dr. Dieter Weber und Klaus Seiffert**
**Patentanwälte Gustav-Freytag-Strasse 25**
**Postfach 6145**
**D-6200 Wiesbaden 1 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 125 508 B1

## Description

This invention relates to a novel method of inhibiting detrusor muscle spasticity and increasing the force of detrusor muscle contraction. Such an invention has many novel applications including but not limited to the treatment of patients suffering from unstable bladder conditions.

The human bladder is composed of four coats; serous, muscular, submucous and mucous. The muscular coat consists of three layers of unstriped muscular fiber: an external layer, composed of fibers having for the most part a longitudinal arrangement; a middle layer in which the fibers are arranged, more or less, in a circular manner; and an internal layer, in which the fibers have a general longitudinal arrangement. In the external longitudinal layer at the sides of the bladder, the muscle fibers are arranged obliquely and intersect one another. This layer is called the detrusor muscle.

Thiphenamil hydrochloride is a class of compounds comprising a di-N-substituted aminoethyl ester of dipenylthioacetic acid having the formula:

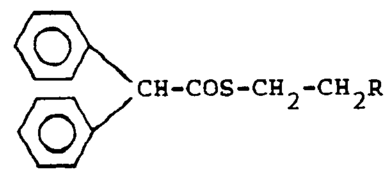

in which R represents a disubstituted amino radical of either the diethylamino group, the morpholino group or the piperidino group.

The prior uses for thiphenamil hydrochloride center around its use as an anti-spasmotic agent in the upper and lower gastro-intestinal tract for pylorospasm, spasm associated with the gallbladder and common bile duct, as well as diarrhea and the irritable bowel syndrome. Prior art uses also include treatment of ureterospasm and bladder irritation.

There have been further reports that thiphenamil hydrochloride has been successfully used for the treatment of bronchospasm.

Thiphenamil hydrochloride is a well-known compound and is described in detail in the US—A—2 390 555. Additional methods of making thiphenamil hydrochloride are described in the US—A—2 510 773.

The term "detrusor instability" generally refers to the condition wherein the bladder spontaneously contracts and empties before the patient is ready.

Generally, the following compounds have been used for the pharmacological treatment of detrusor instability: flavoxate hydrochloride, which is a smooth muscle relaxant, oxybutynin chloride, an anti-cholinergic used for the relief of neurogenic bladder symptoms; and methantheline bromide, an anti-cholinergic and anti-spasmodic drug. For the most part, the above listed and other derivatives of atropine are unpredictable in their effect on bladder instability. In addition to their rather poor successful treatment record, anti-cholinergic compounds produce undesirable side effects such as dryness of the mouth, dilation of the pupils and slowed heartbeat.

In the use of thiphenamil hydrochloride as a smooth muscle relaxant it has generally been assumed that the drug relaxes and inhibits the ability of smooth muscles to contract. This phenomenon has been shown in a number of studies:

1. STUDIES ON THE PHARMACOLOGY OF BETA-LIETHYLAMINOETHYL-DIPHENYLTHIOACETATE, A SYNTHETIC ANTISPASMODIC, Ramsey & Richardson, J. Pharm. & Exp. Th., Feb. 1947, pp,. 131—142.
2. CLINICAL EVALUATION OF TROCINATE, AN ANTISPASMODIC, MacDonald, J. Missouri Medical Association, Sept. 1951, pp. 685—6.
3. USE OF ANTISPASMODICS IN TREATMENT OF SPASTIC URETERITIS, Thackston, et. al., J. Urology, March 1955, pp. 487—493.
4. EFFECT OF A LOCAL SMOOTH MUSCLE ANTAGONIST ON WOUND CONTRACTION, Morton et. al., Surg. Forum, Vol. 23, 1972.
5. MUSCLE DRUG SLOWS WOUND CONTRACTION, Medical World News, March 1974, p. 74A.
6. CONTRACTION OF EXPERIMENTAL WOUNDS I. INHIBITING WOUND CONTRACTION BY USING A TOPICAL SMOOTH MUSCLE ANTAGONIST, Madden et. al., Surgery, July 1974, pp. 8—15.

As a result of these and other studies, persons skilled in the art generally expect the force of the smooth muscle contractions to be greatly diminished after administering thiphenamil hydrochloride.

There has been a need in the pharmaceutical art for a drug which inhibits involuntary detrusor contractions while at the same time increasing the strength of voluntary detrusor contractions.

Thus, it is an important object of the present invention to provide a drug for inhibiting unplanned or premature muscle contraction of the detrusor muscle while at the same time increasing the force of voluntary contraction of the detrusor muscle.

It is yet another, important object of the present invention to provide a drug which is safe for use on human patients, particularly sensitive patients such as glaucoma patients, without subjecting the patient to the undesirable side effects of anti-cholinergic drugs, for example dryness of the mouth, dilation of the pupils and slowed heartbeat.

It is another important object of the present invention to provide a drug for inhibiting unplanned or premature muscle contraction while at the same time increasing the force of the detrusor muscle contraction without leaving or accumulating any foreign substances in the human body tissues.

These objects are met according to the invention by the use of a di-N-substituted aminoethyl ester of diphenylthioacetic acid having the formula:

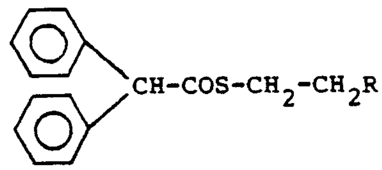

in which R represents a disubstituted amino radical of the group consisting of the diethylamino group, the morpholino group or the piperidino group, for the preparation of a drug for simultaneous inhibition of the detrusor muscle spasticity and increase of the force of contraction of the detrusor muscle.

It is evident from the experimental results which follow that thiphenamil hydrochloride inhibits involuntary detrusor muscle contraction while at the same time increasing the force of voluntary detrusor muscle contractions. Apparently, the combined action of inhibiting detrusor spasticity while increasing the force of detrusor contraction is a unique property of thiphenamil hydrochloride and is an unknown action of any other compound.

The advantage of this effect of thiphenamil hydrochloride is that involuntary detrusor muscle contraction is inhibited thereby giving the patient effective bladder control. Furthermore, because the strength of the detrusor muscle contraction is increased, there is a more complete emptying of the bladder, even in cases where there is a partial obstruction such as in a prostatic obstruction generally encountered in older patients suffering from prostatic hypertrophy.

The experimental results reveal that thiphenamil hydrochloride acts as an effective detrusor spasticity inhibitor and detrusor force of contraction increaser in a dosage range of from about 0.7 to about 11.4 mg/kg of body weight. A preferred dosage is in the range of from about 1.4 to about 5.7 mg/kg of body weight. A still more preferred dosage range is from about 2.8 to about 5.7 mg/kg of body weight.

Thiphenamil hydrochloride can be administered orally, typically in tablets of 100—400 mg or by intravenous injection.

Because thiphenamil hydrochloride slowly hydrolizes in water, it is generally not used as a serum or suspension. It is possible however to encapsulate microspheres of thiphenamil hydrochloride in the form of a liquid suspension for administration to patients.

## Examples

Urodynamic studies were performed on 20 women having a mean age of 28 ± 7 years. These studies incorporated water cystometrogram, UPP, and isometric tests. Control urodynamic testing without use of thiphenamil hydrochloride was undertaken in all subjects. The effects of a single dose of 400 mg and a single dose of 800 mg of thiphenamil hydrochloride given orally were compared with the control value for each subject. Specifically, the measurements taken included bladder capacity, residual volume, maximum detrusor voiding pressure, and isometric contractility during bladder filling and at bladder capacity.

The results of these experiments are shown in Tables I—IV.

The experimental results show no significant increase in bladder capacity and a decrease in the detrusor muscle contractility of from 41.3 ± 15.3 cm $H_2O$ to 37.2 ± 16.0 cm $H_2O$.

The test results further demonstrate that the isomeric detrusor pressure during the filling phase of the bladder significantly increases from 36.1 ± 26.9 cm $H_2O$ to 63.0 ± 27.1 cm $H_2O$.

TABLE I

Stable Detrusor: Urodynamic parameters of the filling and voiding phase of the bladder under control conditions and also with 400 mg and 800 mg thiphenamil hydrochloride.

| | Control | Thiphenamil Hydrochloride (400 mg) | Thiphenamil Hydrochloride (800 mg) |
|---|---|---|---|
| Compliance | 0.003 ± 0.001 | 0.002 ± 0.001 | 0.0025 ± 0.003 |
| V (1st sense) | 281 ± 126 (21) | 314 ± 99 (19) | 298 ± 67.6 (11) |
| V (Urge) | 525 ± 200 (21) | 551 ± 195 (19) | 381 ± 61 (11) |
| V (max) | 552 + 168 (21) | 581 + 155 (19) | 436 ± 165 (11) |
| P (opening) | 29.9 ± 14.0 (21) | 25.4 ± 13.3 (19) | 39.1 ± 12.2 (7) |
| P (max flow) | 36.7 ± 15.8 (21) | 37.9 ± 9.1 (19) | 49.5 ± 14.8 (11) |
| P (voiding) | 41.3 ± 15.5 (21) | 37.2 ± 16 (20) | 65.1 ± 19.1 (11) |
| Q (max) | 16.9 ± 4.6 (21) | 16.7 ± 4.4 (21) | 27.7 ± 9.0 (8) |
| R.U. | 69.3 ± 83.1 (18) | 132.3 ± 121.4 (18) | 95.0 ± 72.2 (9) |

Wherein:

1. Compliance is the compliance of the detrusor muscle in liters/cm $H_2O$.
2. V (1st sense) is the bladder urine volume in ml's at the patient's first sensation to urinate.
3. V (Urge) is the bladder urine volume in ml's at the patient's urge to urinate.
4. V (max) is the maximum bladder urine volume in ml's.
5. P (opening) is the bladder pressure at the patient's initiating urination in cm $H_2O$.
6. P (max flow) is the bladder pressure at maximum urine flow rate in cm $H_2O$.
7. P (voiding) is the bladder voiding pressure in cm $H_2O$.
8. Q (max) is the maximum urine flow rate in ml/sec.
9. R.U. is the residual or left over bladder urine volume in ml's.
10. (n) is the number of measurements taken, i.e. sample size.

TABLE II

Stable Detrusor: Maximum isometric pressure as a function of bladder volume under control conditions and also with 400 mg and 800 mg of thiphenamil hydrochloride.

| | Control | Thiphenamil Hydrochloride (400 mg) | Thiphenamil Hydrochloride (800 mg) |
|---|---|---|---|
| I (100) | 36.1 ± 26.9<br>(10) | 63.0 ± 27.1<br>(13) | 74 ± 34.6<br>(6) |
| I (200) | 37.9 ± 23.4<br>(12) | 51.9 ± 26.0<br>(14) | 65.0 ± 37.8<br>(7) |
| I (300) | 35.9 ± 12.9<br>(8) | 50.3 ± 25.8<br>(12) | 63.4—26.2<br>(7) |
| I (400) | 37.2 ± 17.9<br>(11) | 46.6 ± 19.5<br>(10) | 50.0 ± 27.5<br>(6) |
| I (500) | 36.8 ± 23.7<br>(9) | 30.1 ± 15.6<br>(8) | 34.0 ± 14.4<br>(2) |
| V (1st sense) | 316 ± 147<br>(18) | 358 ± 106<br>(18) | 253.3 ± 96.9<br>(6) |
| V (Urge) | 503 ± 138<br>(18) | 467 ± 94<br>(18) | 385 ± 74.6<br>(7) |
| V (max) | 515 ± 162<br>(13) | 544 ± 151<br>(11) | 425 ± 82<br>(10) |

Wherein:

1. I (100 etc.) is the maximum bladder isometric pressure at a bladder urine volume of 100, 200 etc. ml's in cm $H_2O$.
2. V (1st sense) is the bladder urine volume in ml's at the patient's first sensation to urinate.
3. V (Urge) is the bladder urine volume in ml's at the patient's urge to urinate.
4. V (max) is the maximum bladder urine volume in ml's.
5. (n) is the number of measurements taken, i.e. sample size.

TABLE III

Unstable Detrusor: Urodynamic parameters of the filling and voiding phase of the bladder under control conditiions and also with 400 mg and 800 mg of thiphenamil hydrochloride.

| | Control | Thiphenamil Hydrochloride (400 mg) | (800 mg) |
|---|---|---|---|
| Compliance | 0.003 ± 0.001 | 0.002 ± 0.001 | 0.0027 ± 0.0004 |
| V (1st sense) | 217 ± 202<br>(4) | 270 ± 99<br>(3) | 275 ± 35<br>(3) |
| V (Urge) | 345 ± 209 | 345 ± 191 | 325 ± 35<br>(2) |
| V (max) | 398 ± 171<br>(3) | 440 ± 57<br>(2) | 499 ± 177<br>(3) |
| P (opening) | 38 ± 17 | 30 ± 12<br>(4) | 44 ± 11.3<br>(2) |
| P (maxflow) | 44.3 ± 3.1<br>(3) | 44.0 ± 4.4<br>(3) | 55.3 ± 7.0<br>(3) |
| P (voiding) | 53 ± 18<br>(4) | 47 ± 7<br>(4) | 52.7 ± 6.4<br>(3) |
| Q (max) | 18.2 ± 1.2 | 15 ± 1.9 | 18.0 ± 1.8<br>(3) |
| R.U. | 18 ± 22<br>(3) | 140 ± 85<br>(2) | 27.5 ± 3.5<br>(3) |

Wherein:

1. Compliance is the compliance of the detrusor muscle in liters/cm $H_2O$.
2. V (1st sense) is the bladder urine volume in ml's at the patient's first sensation to urinate.
3. V (Urge) is the bladder urine volume in ml's at the patient's urge to urinate.
4. V (max) is the maximum bladder urine volume in ml's.
5. P (opening) is the bladder pressure at the patient's initiating urination in cm $H_2O$.
6. P (max flow) is the bladder pressure at maximum urine flow rate in cm $H_2O$.
7. P (voiding) is the bladder voiding pressure in cm $H_2O$.
8. Q (max) is the maximum urine flow rate in ml/sec.
9. R.U. is the residual or left over bladder urine volume in ml's.
10. (n) is the number of measurements taken, i.e. sample size.

TABLE IV

Unstable detrusor: Maximum isometric pressure as a function of bladder volume under control conditions and also with 400 mg and 800 mg of thiphenamil hydrochloride.

| | Control | Thiphenamil Hydrochloride (400 mg) | (800 mg) |
|---|---|---|---|
| I (100) | 32 ± 13 (3) | 73 ± 39 (14) | 78 ± 53 |
| I (200) | 42 ± 35 (4) | 44 ± 14 (3) | 62 ± 57 |
| I (300) | 35 ± 13 | 75 ± 5 (2) | 96 (1) |
| V (1st sense) | 217 ± 143 (4) | 270 + 99 (4) | |
| V (urge) | 320 ± 170 (4) | 355 ± 53 (4) | 423 ± 27.5 |

Wherein:

1. Compliance is the compliance of the detrusor muscle in liters/cm $H_2O$.
2. V (1st sense) is the bladder urine volume in ml's at the patient's first sensation to urinate.
3. V (Urge) is the bladder urine volume in ml's at the patient's urge to urinate.
4. V (max) is the maximum bladder urine volume in ml's.
5. P (opening) is the bladder pressure at the patient's initiating urination in cm $H_2O$.
6. P (max flow) is the bladder pressure at maximum urine flow rate in cm $H_2O$.
7. P (voiding) is the bladder voiding pressure in cm $H_2O$.
8. Q (max) is the maximum urine flow rate in ml/sec.
9. R.U. is the residual or left over bladder urine volume in ml's.
10. (n) is the number of measurements taken, i.e. sample size.

**Claim**

Use of a di-N-substituted aminoethyl ester of diphenylthioacetic acid having the formula:

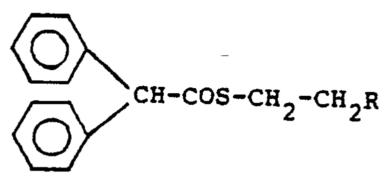

in which R represents the diethylamino group, the morpholino group or the piperidino group, for the preparation of a drug for simultaneous inhibition of the detrusor muscle spasticity and increase of the force of contraction of the detrusor muscle.

**Patentanspruch**

Verwendung eines di-N-substituierten Aminoethylesters von Diphenylthioessigsäure der Formel

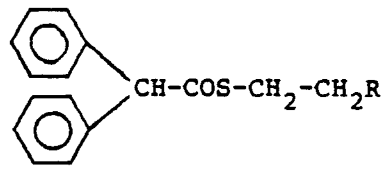

worin R die Diethylaminogruppe, die Morpholinogruppe oder die Piperidinogruppe bedeutet, zur

Herstellung eines Arzneimittels für gleichzeitige Hemmung der Detrusormuskel-Spastizität und Steigerung der Kontraktionsstärke des Detrusormuskels.

**Revendication**

Utilisation d'un ester (amino di-N-substitué)-éthylique de l'acide diphénylthioacétique, de formule:

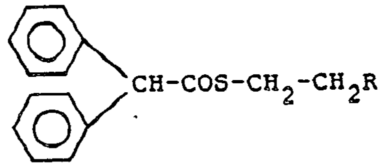

dans laquelle R représente le groupe diéthylamino, le groupe morpholino ou le groupe pipéridino, pour la préparation d'un médicament permettant, d'une façon simultanée, d'inhiber la spasticité du muscle détrusor et d'augmenter la force de contraction du muscle détrusor.